# DEMANDE DE BREVET EUROPEEN

(11) **EP 0 951 869 A1**
(43) Date de publication de la demande: **27.10.1999**
(21) Numéro de dépôt: 99420099.6
(22) Date de dépôt: 21.04.1999
(51) Int. Cl.: A61B 17/04, F16B 25/10

(54) **Ancre de suture vissée**

(30) Priorité: 21.04.1998 FR 9805205
(71) Demandeur: Tornier SA, 38330 Saint Ismier (FR)
(72) Inventeur: Tornier, Alain, 38330 Saint Ismier (FR)
(74) Mandataire: Schmitt, John

(57) **Abrégé**

L'ancre de suture comporte, d'une part une tête de manoeuvre (2) qui se prolonge par un corps fileté (3) et une âme (4) pour la réinsertion de tissus mous contre l'os, et d'autre part à l'opposé de la tête de manoeuvre (2) une extrémité pointue (6) qui est constituée par le profil du filet (22) du filetage (5) pour permettre d'agripper les tissus mous.

## Description

La présente invention est relative à une ancre de suture vissée comportant une tête de manoeuvre qui se prolonge par un corps fileté pour son introduction dans l'os d'un patient.

L'ancre de suture suivant la présente invention comporte une tête de manoeuvre permettant la retenue des fils de suture pour pouvoir, par exemple, rattacher des tissus mous tels que des ligaments, des tendons à l'os, et principalement pour la réparation de la coiffe des rotateurs et des lésions de Bankart.

On connaît d'après le brevet européen 0686373, une ancre de suture comportant une tige présentant une extrémité distale et une extrémité proximale. Un filetage fait sailli de la tige et s'étend hélicoïdalement de l'extrémité proximale à l'extrémité distale.

Un orifice transversal traverse la tige et le filetage à proximité de l'extrémité proximale. L'orifice transversal reçoit une suture qui foumit une double extrémité de suture pour faciliter la fixation à des tissus mous.

La tige est effilée d'un diamètre plus grand côté proximal, à un diamètre plus petit côté distal, tandis que le grand diamètre du filetage reste constant sur une grande partie de sa longueur. L'ancrage de suture comporte une partie de commande qui, de préférence, comporte une rainure destinée à guider la suture depuis l'orifice transversal jusqu'à l'extrémité libre de la partie de commande. L'ancrage de suture comprend un dispositif d'entraînement, qui possède une partie d'engagement destinée à venir coopérer avec la partie de commande, et une rainure. Lorsque la partie d'engagement vient coopérer avec la partie de commande, les deux rainures sont alignées pour former un passage fermé, destiné à guider la suture, lorsque la suture traverse la partie de commande.

On remarque que le pas du filet n'est pas adapté pour un ancrage optimal dans l'os, tandis que l'âme de la tige possède un diamètre relativement important, impliquant un rapport entre le diamètre de l'âme et le diamètre du filetage qui rend impossible l'insertion directe de l'ancre de suture dans le tissu osseux, sans un pré-perçage ou taraudage.

On note également que la tige comporte un dispositif de retenue qui n'est pas prévu pour sertir définitivement les fils de suture.

C'est à ces inconvénients qu'entend plus particulièrement remédier la présente invention.

L'ancre de suture suivant la présente invention comporte une tête de manoeuvre qui se prolonge par un corps fileté et une âme, tandis qu'à l'opposé de ladite tête est prévue une extrémité pointue qui est constituée par le profil du filet d'un filetage pour permettre d'agripper les tissus mous.

L'ancre de suture suivant la présente invention comporte une extrémité pointue qui est constituée par le profil du filet du filetage et qui s'étend dans une direction sensiblement perpendiculaire à celle de l'axe longitudinal portant l'âme du corps fileté.

L'ancre de suture suivant la présente invention comporte une tête de manoeuvre qui se prolonge par un corps fileté présentant une âme à profil cylindro-conique et un filet de conicité de 15 à 20 degrés, tandis que le rapport entre le diamètre de l'âme et le diamètre extérieur du filetage est compris entre 1,5 et 2,5 pour permettre une insertion de l'ancre dans le tissu osseux sans pré-perçage.

L'ancre de suture suivant la présente invention comporte un corps dont le pas du filet est compris entre 1,8 et 2,2 millimètres.

L'ancre de suture suivant la présente invention comporte un corps fileté dont le diamètre de l'âme est compris entre 1 et 3 millimètres.

L'ancre de suture suivant la présente invention comporte un corps fileté dont la naissance du filetage sur l'âme au niveau de l'extrémité pointue, est conique.

L'ancre de suture suivant la présente invention comprend une tête de manoeuvre à profil hexagonal percé d'un trou pour le passage des fils de suture.

L'ancre de suture suivant la présente invention comprend une tête de manoeuvre pourvue d'un dispositif de sertissage présentant des moyens de fixation qui sont constitués de deux languettes susceptibles d'être déformées plastiquement par rapport au corps fileté, lesdites languettes délimitant avant déformation un espace oblong qui est traversé par au moins un fil de suture, ledit fil de suture étant serti entre lesdites languettes après déformation de ces dernières sous un effort de traction.

L'ancre de suture suivant la présente invention comprend un dispositif de sertissage constitué d'une face d'appui solidaire des languettes de sertissage et percée d'un alésage qui débouche entre lesdites languettes, et d'une partie cylindrique disposée à l'opposé de la face d'appui et dans le prolongement des languettes de sertissage, ladite partie cylindrique étant percée d'un trou borgne fileté prévu pour recevoir une tige filetée d'un ancillaire pour déformer sous un effort de traction les languettes suivant une direction sensiblement perpendiculaire à l'axe longitudinal du corps fileté pour la fixation d'au moins un fil de suture.

La description qui va suivre en regard des dessins annexés, donnés à titre d'exemples non limitatifs, permettra de mieux comprendre l'invention, les caractéristiques qu'elle présente et les avantages qu'elle est susceptible de procurer :

Figure 1 est une vue illustrant l'ancre de suture suivant la présente invention.

Figure 2 est une vue montrant une première variante de l'ancre de suture suivant la présente invention.

Figure 3 est une vue représentant une seconde variante de l'ancre de suture suivant la présente invention.

Figure 4 est une vue illustrant la tête de manoeuvre pourvue d'un dispositif de sertissage pour la retenue des fils de suture sur l'ancre de suture suivant la présente invention.

Figure 5 est une vue montrant le dispositif de sertissage avant déformation coopérant avec un ancillaire.

Figure 6 est une vue représentant le dispositif de sertissage après déformation pour la retenue des fils de suture sur l'ancre de suture suivant la présente invention.

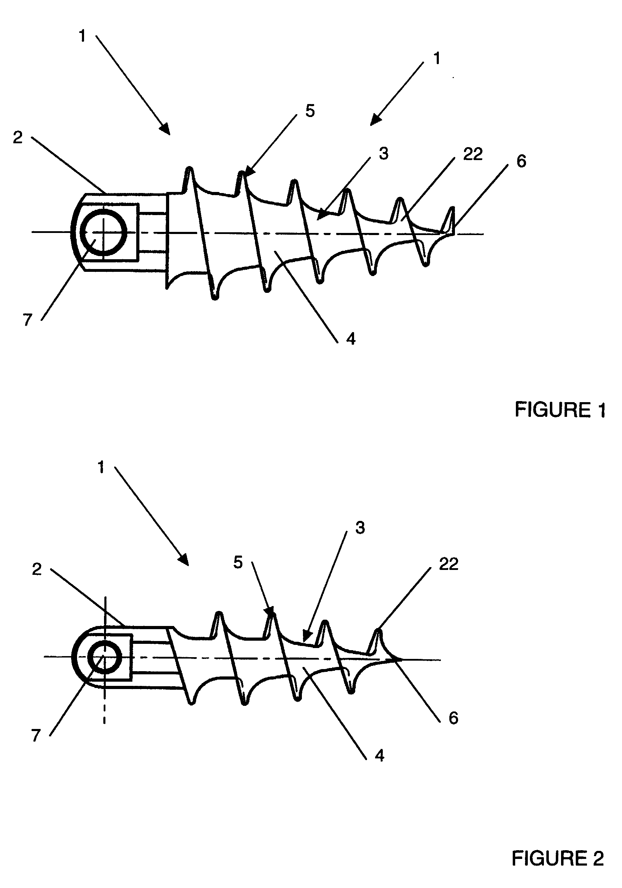

On a montré en figures 1 à 3 une ancre de suture 1 comportant une tête de manoeuvre 2 se prolongeant par un corps fileté 3 susceptible de pénétrer dans le tissu osseux pour son ancrage.

Le corps fileté 3 comprend une âme 4 à profil cylindro-conique dont le diamètre est compris entre 1 et 3 millimètres et un filetage 5 dont la conicité est de 15 à 20 degrés. Le rapport entre le diamètre de l'âme 4 et le diamètre extérieur du filetage 5 est compris entre 1,5 et 2,5 pour permettre une insertion de l'ancre 1 dans le tissu osseux sans pré-perçage.

Le corps fileté 3 présente à l'opposé de la tête de manoeuvre 2 une extrémité pointue 6 qui est constituée par le profil du filet 22 du filetage 5 pour permettre d'agripper les tissus mous. La forme de la pointe de l'extrémité 6 est créée par le démarrage immédiat du filet 22.

On constate que l'extrémité pointue 6 constituée par le profil du filet 22 du filetage 5, s'étend dans une direction sensiblement perpendiculaire à celle de l'axe longitudinal portant l'âme 4 de l'ancre de suture 1.

Le pas du filetage 5 du corps 3 est compris entre 1,8 et 2,2 millimètres pour éviter de détériorer le tissus osseux lors de la mise en place de l'ancre de suture 1.

L'ancre de suture 1 comporte un corps fileté 3 dont la naissance du filetage 5 sur l'âme 4 au niveau de l'extrémité pointue 6, est conique.

Le profil du filet 22 est pointu et tranchant pour faciliter l'insertion de l'ancre de suture 1 dans le tissu osseux.

La tête de manoeuvre 2 présente un profil hexagonal permettant l'entraînement en rotation de l'ancre de suture 1 par l'intermédiaire d'un outil tel qu'un tournevis pour son introduction dans le tissu osseux.

Les dimensions de l'hexagone sont prévues pour recevoir un tournevis dont le diamètre extérieur est inférieur à celui de l'ancre de suture 1 pour pouvoir traverser les tissus mous à réinsérer avant de trouver l'ancrage dans le tissus osseux.

La tête de manoeuvre 2 est percée d'un alésage 7 permettant le passage des fils de suture 8 pour la fixation des tissus mous.

L'ensemble des caractéristiques ci-dessus permet le vissage de l'ancre de suture 1 sans être obligé de pratiquer un pré-perçage dans l'os. Egalement, elles autorisent l'insertion de l'ancre de suture 1 sous un angle éloigné de l'axe perpendiculaire à la surface osseuse.

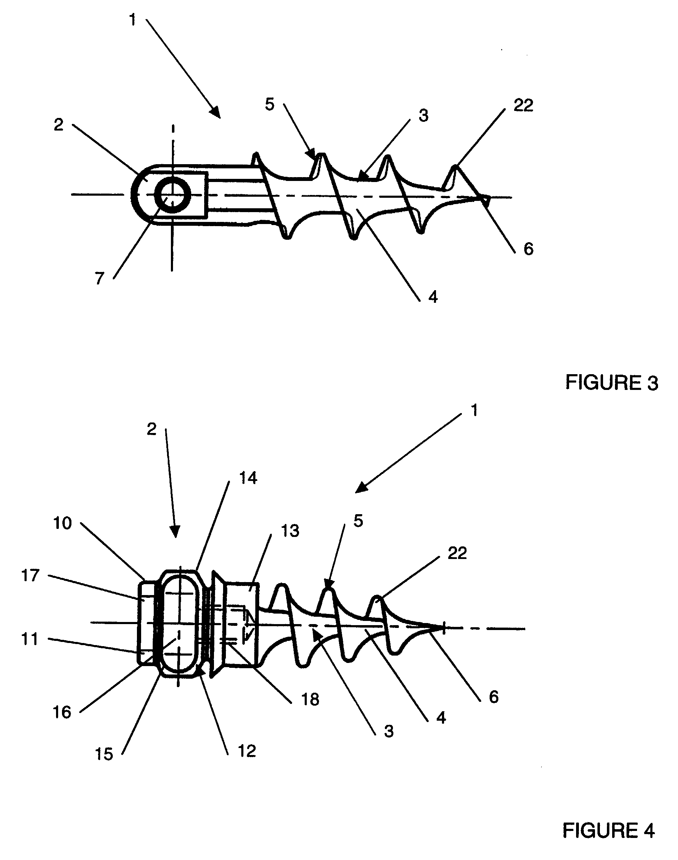

On a montré en figure 4 l'ancre de suture 1 dont la tête de manoeuvre 2 présente un dispositif de sertissage 9 comportant dans le prolongement du corps fileté 3 une partie cylindrique creuse 10 susceptible de se déformer plastiquement.

La partie cylindrique 10 est constituée à l'une de ses extrémités d'une face d'appui 11 solidaire de moyens de fixation 12 pour le sertissage d'au moins un fil de suture 8 permettant au chirurgien de ligaturer des tissus mous contre l'os.

La partie cylindrique 10 comprend à l'opposé de la face d'appui 11, et dans le prolongement des moyens de fixation 12, une portion de cylindrique 13 permettant le raccordement du dispositif de sertissage 9 de la tête 2 avec le corps fileté 3.

Les moyens de fixation 12 sont constitués de deux languettes de sertissage 14, 15 qui sont disposées parallèlement à l'axe longitudinal XX' du corps fileté 3.

Les languettes 14, 15 délimitent, avant déformation plastique, un espace oblong 16 qui permet le passage, par le chirurgien à l'extérieur ou sur le site opératoire, d'au moins un fil de suture 8.

L'espace oblong 16 est positionné de manière que sa direction de plus grande longueur soit perpendiculaire à l'axe longitudinal XX' du corps fileté 3 de l'ancre de suture 1.

La face d'appui 11, réunissant les languettes 14 et 15 à l'opposé de la portion de cylindrique 13, est percée d'un alésage 17, porté par l'axe XX', et qui débouche, d'une part à l'extérieur de la partie cylindrique 10, et d'autre part entre lesdites languettes.

Egalement, la portion cylindrique 13 présente dans sa partie interne un trou borgne fileté 18 qui débouche entre les languettes 14, 15, et qui est porté par le même axe longitudinal XX' que celui de l'alésage 17. En outre, le diamètre de l'alésage 17 est prévu plus grand que celui du trou fileté 18.

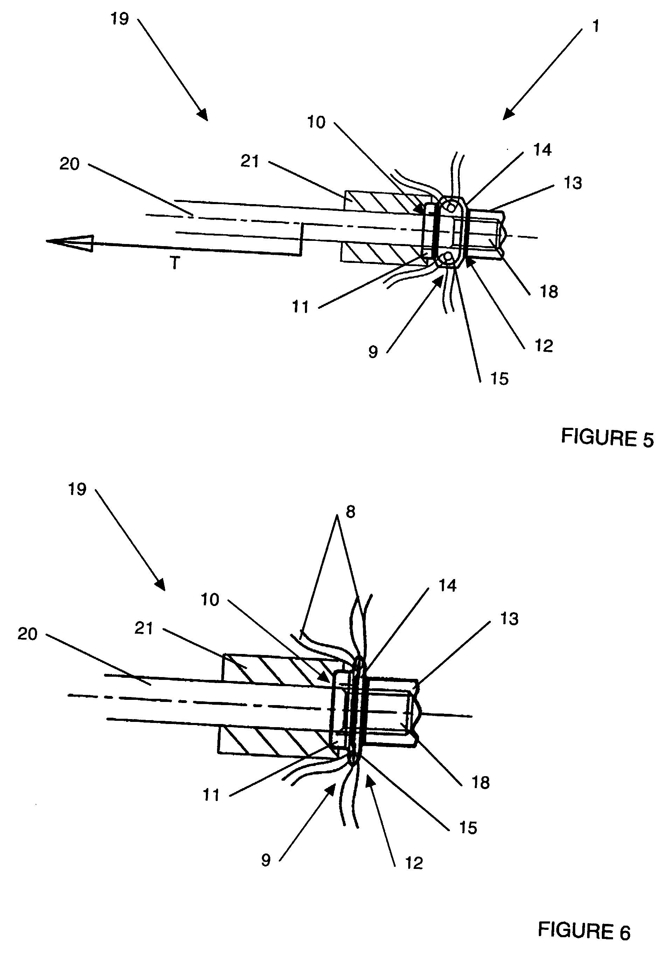

En figures 5 et 6 on a illustré le sertissage des fils de suture 8 sur l'ancre de suture 1 par l'intermédiaire du dispositif de sertissage 10 de la tête de manoeuvre 2.

Le dispositif de sertissage 9 coopère avec un ancillaire 19 de mise en place qui est constitué, par exemple, d'une tige 20 qui traverse la partie cylindrique 10 pour venir se visser dans le trou borgne 18 de la portion cylindrique 13. La tige 20 est solidaire d'un embout 21 qui vient prendre appui contre la face 11 de la partie cylindrique 10.

Un effort de traction T est appliqué sur la tige 20 de l'ancillaire 19 afin de déformer les languettes 14 et 15, des moyens de fixation 12, suivant une direction sensiblement perpendiculaire à l'axe longitudinal XX' du corps fileté 3 pour la fixation d'au moins un fil de suture 8 (figure 5).

La déformation des languettes 14, 15 permet de réduire l'espace oblong 16 de manière à bloquer dans une position tendue les fils de suture 8 sur le corps fileté 3 de l'ancre de suture 1 (figure 6).

Les languettes 14 et 15 sont conformées pour ne pas couper les fils de suture 8 lors du sertissage de ces demiers sur le corps fileté 3.

On note que les languettes 14, 15 peuvent présenter un autre profil extérieur sans pour autant changer l'objet de la présente invention.

## Revendications

1. Ancre de suture comportant une tête de manoeuvre (2) qui se prolonge par un corps fileté (3) et une âme (4) pour la réinsertion de tissus mous contre l'os, **caractérisé en ce qu'**elle comporte à l'opposé de la tête de manoeuvre (2) une extrémité pointue (6) qui est constituée par le profil du filet (22) du filetage (5) pour permettre d'agripper les tissus mous.

2. Ancre de suture suivant la revendication 1, **caractérisée en ce que** l'extrémité pointue (6), constituée par le profil du filet (22) du filetage (5), s'étend dans une direction sensiblement perpendiculaire à celle de l'axe longitudinal portant l'âme (4) du corps fileté (3).

3. Ancre de suture suivant la revendication 1, **caractérisée en ce qu'**elle comporte une tête de manoeuvre (2) qui se prolonge par un corps fileté (3) présentant une âme (4) à profil cylindro-conique et un filetage (5) de conicité de 15 à 20 degrés, tandis que le rapport entre le diamètre de l'âme (4) et le diamètre extérieur du filetage (5) est compris entre 1,5 et 2,5 pour permettre une insertion de l'ancre (1) dans le tissu osseux sans pré-perçage.

4. Ancre de suture suivant la revendication 1, **caractérisée en qu'**elle comporte un corps fileté (3) dont le pas du filetage (5) est compris entre 1,8 et 2,2 millimètres.

5. Ancre de suture suivant la revendication 1, **caractérisée en ce qu'**elle comporte un corps fileté (3) dont le diamètre de l'âme (4) est compris entre 1 et 3 millimètres.

6. Ancre de suture suivant la revendication 1, **caractérisée en ce qu'**elle comporte un corps fileté (3) dont la naissance du filetage (5) sur l'âme (4) au niveau de l'extrémité pointue, est conique.

7. Ancre de suture suivant la revendication 1, **caractérisée en ce que** le profil du filet (22) est pointu et tranchant.

8. Ancre de suture suivant la revendication 1, **caractérisée en ce qu'**elle comprend une tête de manoeuvre (2) à profil hexagonal percée d'un alésage (7) pour le passage des fils de suture (8).

9. Ancre de suture suivant la revendication 1, **caractérisé en ce qu'**elle comprend une tête de manoeuvre (2) pourvue d'un dispositif de sertissage (9) présentant des moyens de fixation (12) qui sont constitués de deux languettes (14, 15) susceptibles d'être déformées plastiquement par rapport au corps fileté (3), lesdites languettes délimitant avant déformation un espace oblong (16) qui est traversé par au moins un fil de suture (8), ledit fil de suture étant serti entre lesdites languettes après déformation de ces dernières sous un effort de traction (T).

10. Ancre de suture suivant la revendication 9, **caractérisée en ce que** le dispositif de sertissage (9) est constitué d'une face d'appui (11) solidaire des languettes de sertissage (14, 15), d'un alésage (17) qui débouche entre lesdites languettes, et d'une portion cylindrique (13) disposée à l'opposé de la face d'appui (11) et dans le prolongement des languettes de sertissage (14, 15), ladite portion cylindrique étant percée d'un trou borgne fileté (18) prévu pour recevoir une tige filetée (20) d'un ancillaire (19) pour déformer, sous un effort de traction (T), les languettes (14, 15), suivant une direction sensiblement perpendiculaire à l'axe longitudinal XX' du corps fileté (3) pour la fixation d'au moins un fil de suture (8).
